# EUROPEAN PATENT APPLICATION

(11) **EP 2 037 242 A1**
(43) Date of publication of application: **18.03.2009**
(21) Application number: 07017768.8
(22) Date of filing: 11.09.2007
(51) Int. Cl.: G01J 3/02, G01N 33/36, G01N 21/29

(54) **Colour assessment cabinet**

(71) Applicant: Verivide Ltd, Warrrens Business Park Enderby Leicestershire LE19 4SG (GB)
(72) Inventor: Dakin, John c/o Verivide Ltd, Leicester LE19 4SG (GB)
(74) Representative: Smith, Peter James

(57) **Abstract**

The invention provides a colour assessment cabinet comprising an interior chamber (2) and a light source (14) for illuminating the chamber (2). The cabinet further comprises a fan (28) for drawing ambient air through the chamber (2). The cabinet can thereby be used not only for colour assessment of a sample but also for conditioning the sample prior to assessment when the ambient air has suitable temperature and humidity, such as in a laboratory environment. Use of a common light source in the conditioning and assessment steps provides economy and avoids the risk of inconsistent assessment due to different light sources producing different photochromic responses in the sample.

## Description

### Technical field of the invention

The invention relates to the field of colour assessment of samples such as textiles and to the conditioning of such samples prior to assessment in order to ensure consistent results.

### Background of the invention

Colour assessment is important for determining the appearance of samples under known lighting conditions in a diverse range of industries such as textiles, printed media and paints. The assessment may be to check the quality of a single sample under prescribed lighting conditions, for example a photograph in a magazine, or may be to compare different samples, for example to ensure that two samples of dyed fabric that are to be used in a garment match one another reliably in different lighting conditions. Assessment includes assessing the colour by eye or measuring it with an instrument such as a camera or spectrometer. It is essential that the conditions under which the samples are assessed should be standardized, for example so that a supplier and a purchaser can compare sample products consistently using their respective apparatus.

Colour assessment in carried out in a specialized colour assessment cabinet that provides carefully controlled lighting conditions using one or more light sources to imitate a reference illuminant for the assessment. The reference illuminant may represent natural daylight, typical store lighting or other standard light sources, as defined by an international body such as the Commission Internationale de l'Eclairage (CIE) or specified by a commercial or industrial body. An illuminant is defined by its spectral power distribution, i.e. the intensity of the light at each wavelength over a specified range. For example, the CIE has defined a particular spectral power distribution D65, which is described as "average north sky daylight".

The measured colour of the sample may depend not only on the incident light but also on the condition of the sample. For example, dyed textiles can appear to be different colours under different conditions of temperature and humidity. Certain dyes such as azo dyes can also be photochromic, meaning that they undergo a colour change when excited by incident light of certain wavelengths. The colour change may be reversed instantly when the incident light is removed, or may persist for varying lengths of time. The consequence is that the appearance of the sample also depends on the recent history of its illumination. The degree of photochromism may also be influenced by physical conditions such as humidity and temperature.

For these reasons, in order for the colour assessment of a sample to give consistent results it is conditioned by holding it under standard conditions of temperature, humidity and illumination for a predetermined period of time before the colour assessment is carried out. For example, it may be held for 30 minutes at 20°C and 65% humidity, while illuminated with a daylight source. This conditioning is done in a specialized conditioning cabinet, with temperature and humidity control and adapted to include a light source such as an off-the-shelf daylight bulb. Although the cabinet may be able to hold multiple samples for conditioning in parallel, it is an expensive and bulky item of equipment. Typically, the daylight bulb used in such cabinets is not a particularly close match to the reference illuminant used for colour assessment. While an approximate match may be sufficient for conditioning the sample in many cases, it does give rise to the possibility that the change in lighting on transferring the sample to a colour assessment cabinet may lead to photochromic effects and inconsistent results. Moreover, in order to insulate the interior of the conditioning cabinet from the bulb, it is usual to insert a layer of transparent insulating material such as multi-wall polycarbonate in between, which can further distort the spectrum of light that reaches the sample.

As soon as the conditioned sample is removed from the conditioning cabinet, there is a risk that it may begin to revert to its unconditioned state. Therefore a maximum delay of, for example, five minutes is prescribed during which the transfer, set up and assessment of the sample must be carried out.

### Summary of the invention

The invention provides a colour assessment cabinet as defined in claim 1.

The invention further provides a method of colour assessment of a sample as defined in claim 9.

Preferred but non-essential features of the invention are defined in the dependent claims.

The invention depends upon the realization that in many cases colour assessment of samples is carried out in laboratories in which the temperature and humidity are already carefully controlled to values suitable for conditioning the samples. It is therefore necessary only to provide suitable illumination in order to complete the set of conditions required. That can be done by making use of the light sources that are already present in a colour assessment cabinet, with suitable adaptations to ensure that the temperature and humidity inside the cabinet match the ambient conditions in the laboratory. This has the advantage that a specialized, separate conditioning cabinet can be dispensed with. Moreover, because the light source in the colour assessment cabinet can be used for both the conditioning and assessment steps, a perfect match is guaranteed. It may also be possible to assess the sample while conditioning continues, thereby avoiding any deterioration in its condition caused by removing it from the controlled environment of the cabinet.

It may not be necessary for the temperature and humidity of the ambient air to match precisely those prescribed for the conditioning process. For example, it has been found that at humidity levels above the prescribed 65% cotton fabrics do not absorb any additional moisture so it would be possible to condition such fabrics successfully even in those conditions.

### Drawings

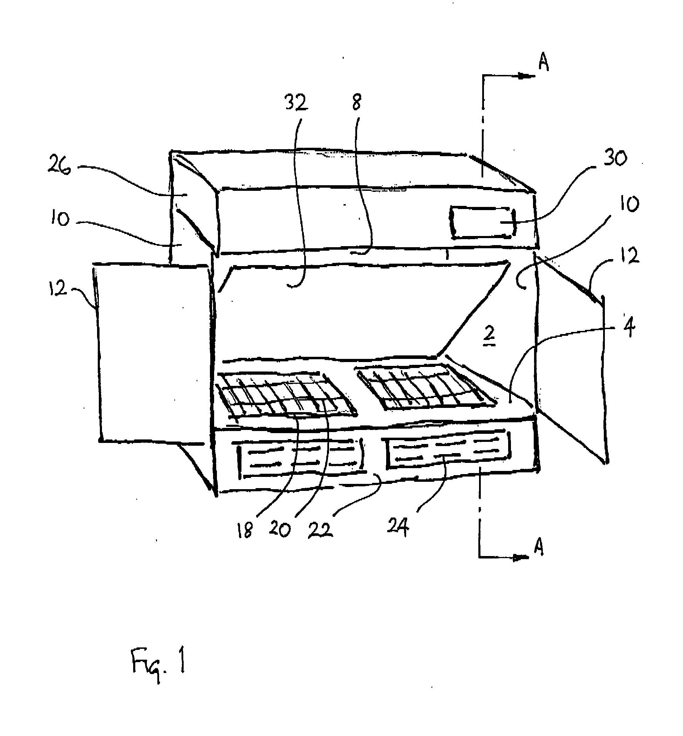

Figure 1 is a perspective view of a colour assessment cabinet in accordance with the invention.
Figure 2 is a sectional side view of the cabinet on line A-A of Figure 1.

### Description of the preferred embodiments of the invention

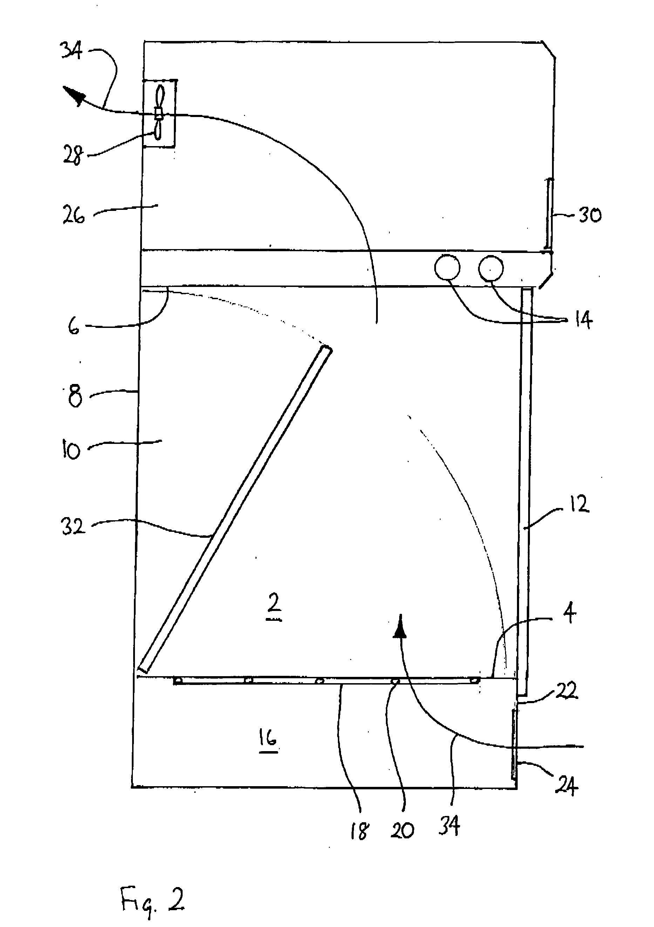

The colour assessment cabinet shown in the drawings has an interior chamber 2 defined by a floor 4, ceiling 6, back wall 8 and two side walls 10. The front of the chamber 2 is defined by a pair of hinged doors 12 that can be opened to place samples in the chamber 2 or to view the samples in the chamber 2; and can be closed to exclude ambient light from the chamber 2. Set into the ceiling 6 of the chamber 2 are one or more light sources 14, shown schematically in Figure 2, such as fluorescent lamps, incandescent lamps or LED clusters of known spectral power distribution. Typically, each light source 14 is designed to illuminate the interior of the chamber 2 with a spectrum that matches as closely as possible a specific reference illuminant. However, multiple light sources 14 may be used in combination to create different spectra. In designing the light sources 14, allowance may be made for changes caused by reflection of the illumination from the interior surfaces of the chamber 2 before it reaches the samples.

The floor 4 of the chamber 2 is raised above the base of the cabinet to create a manifold 16 beneath the floor 4. Apertures in the floor 4 allow air to flow from the manifold 16 into the chamber 2. In the embodiment shown, the apertures take the form of two large openings 18, each covered by a grille 20. Alternatively, they could take the form of multiple smaller holes formed in the floor 4 itself. Many other alternatives will immediately present themselves to the reader and are intended to fall within the scope of the present invention. A lower front wall 22 of the cabinet is pierced by vents 24 that allow air to flow from the exterior of the cabinet into the manifold 16.

Above the ceiling 6 of the chamber 2, the cabinet includes a housing 26 for the power supply, switching and control circuits (not shown). The housing 26 also contains a fan 28 that operates to draw air out of the chamber 2 through apertures (not shown) in the ceiling 6 and expel it to the surroundings. A control panel 30 on the front of the housing 26 allows a user to switch between the various light sources 14, to switch the fan on or off, and to set other operating parameters of the cabinet.

Within the chamber 2, a panel 32 is mounted so that it can pivot about a hinge line close to the junction between the back wall 8 and the floor 4. In a first position the panel 32 lies flat against the floor 4, concealing the openings 18 and preventing air from flowing into the chamber 2. In a second position the panel 32 rests against the back wall 8, revealing the openings 18 and allowing air to flow into the chamber 2. In Figure 2, the panel 32 is illustrated between these two positions.

Other arrangements for concealing the openings 18 can readily be conceived. The panel 32 need not be hinged inside the chamber but could be a separate, removable component, which would allow the samples to be arranged on it before being placed in the chamber 2. In the embodiment shown with two openings 18, there might be two panels hinged against the side walls 10, each of which folds down to cover a respective opening 18. It might be possible to prop the panel in a position rising from front to back of the chamber 2, thereby presenting an inclined surface on which to display samples for viewing through the doors 12. Means other than a hinged panel could also be used to close the openings 18, such as sliding panels built into the floor 4. Depending on the form of the apertures in the floor 4, in some embodiments of the invention it may not be necessary to conceal them at all.

When the cabinet is operated in a conditioning mode, the hinged panel 32 is raised to reveal the openings 18, and samples such as textile samples are placed on the grilles 20. The doors 12 are closed to exclude ambient light from the chamber 2 and a suitable light source 14 for conditioning - typically a daylight source - is switched on to illuminate the samples. The fan 28 is also switched on to expel air from the chamber 2 and cause replacement air to be drawn in from the surroundings through vents 24, manifold 16 and openings 18, as shown by arrows 34. The ambient air thereby passes over and around the samples on the grilles 20 to condition them to substantially the same temperature and humidity as the ambient air. By mounting the fan 28 above the chamber 2, any air that may be heated by proximity to the light sources 14 is drawn out of the chamber 2 without coming into contact with the samples. It is arranged that the air exits from the rear of the cabinet in order to reduce the likelihood that it may be recirculated into the chamber 2 via the vents 24.

Preferably, the cabinet has a preset conditioning setting, which can be chosen from the control panel 30 to operate the correct light source and the fan together. The control panel 30 may conveniently provide a timer display to count down the remaining conditioning time and may also display measurements of temperature and humidity inside the chamber 2.

When the conditioning time has elapsed, the fan 28 may be switched off, the samples removed from the grilles 20 and the hinge panel 32 lowered to conceal the openings 18. The samples may then be replaced on the floor 4 of the chamber 2 or on a suitable display stand (not shown) for viewing by eye through the open doors 12, after selecting a suitable light source 14 or combination of sources to illuminate them. Alternatively, the samples may be viewed with the doors 12 closed by using a camera (not shown) mounted in the ceiling 6 of the cabinet. In this case, the camera can be operated while the cabinet is still in conditioning mode, or at least without opening the doors 12, so that the sample remains in perfect condition during colour assessment. For assessment of the samples by eye, it is possible to provide each door 12 with a viewing slit (not shown) that can be covered to exclude ambient light during conditioning but can be then opened to view the sample *in situ* with a minimum of disturbance to its condition. The correct assessment of some samples requires them to be viewed at a specified angle and the provision of viewing slits in the doors 12 usefully defmes the correct viewing position in such cases.

Conveniently, the same light source 14 may be used for the conditioning and colour assessment steps, which ensures that there will be no change in illumination after conditioning that might lead to inconsistent colour assessment as a result of photochromism. However, it may be possible to shorten the conditioning process by using an alternative light source or an additional secondary light source such as LEDs to boost wavelengths to which the sample dyes are particularly sensitive, thereby accelerating their photochromic response. Preferably, the secondary light source is tunable to match the sensitive wavelengths of different types of dyes.

## Claims

1. A colour assessment cabinet comprising:
a chamber (2) in the interior of the cabinet;
a light source (14) for illuminating the chamber (2);
means (28) for drawing ambient air through the chamber (2).

2. A colour assessment cabinet according to claim 1, wherein the chamber (2) has a floor perforated by at least one aperture (18) through which the ambient air enters the chamber (2).

3. A colour assessment cabinet according to claim 2, further comprising a grille (20) across the aperture (18).

4. A colour assessment cabinet according to claim 2 or claim 3, further comprising means for selectively covering the aperture (18).

5. A colour assessment cabinet according to claim 4, wherein means for covering the aperture (18) comprises a panel (32) mounted in the chamber (2) so as to be pivotable between a position adjacent to a wall (8) of the chamber and a position covering the aperture (18).

6. A colour assessment cabinet according to any preceding claim, wherein the means for drawing ambient air through the chamber (2) is a fan (28) arranged to withdraw air from the chamber (2).

7. A colour assessment cabinet according to any preceding claim, comprising a light source (14) matched to a daylight standard light source.

8. A colour assessment cabinet according to claim 7, further comprising a secondary light source (14) that emits a spectrum suitable for accelerating photochromism in a sample in the chamber (2).

9. A method of colour assessment of a sample, comprising:
placing the sample in a chamber (2) in a colour assessment cabinet;
for a predetermined time, conditioning the sample in the chamber (2) by illuminating the sample with a light source (14) and drawing ambient air through the chamber (2); and
at the end of the predetermined time, illuminating the sample in the chamber (2) with a light source (14) to assess the colour of the sample.

10. A method according to claim 9, wherein the same light source (14) is used to illuminate the sample during the conditioning and colour assessment steps.

11. A method according to claim 9 or claim 10, wherein ambient air continues to be drawn through the chamber (2) during the colour assessment step.
